# EUROPEAN PATENT APPLICATION

(11) **EP 3 395 241 A1**
(43) Date of publication of application: **31.10.2018**
(21) Application number: 18165833.7
(22) Date of filing: 05.04.2018
(51) Int. Cl.: A61B 5/01, A61B 5/00, A61B 18/14, A61B 18/00

(54) **ESOPHAGEAL RETRACTOR WITH SENSOR**

(30) Priority: 28.04.2017 US 201762492035 P; 01.03.2018 US 201815909908
(71) Applicant: Rex Medical, L.P., Conshohocken, PA 19428 (US)
(72) Inventor: McGuckin Jr., James F, Radnor, PA Pennsylvania 19087 (US)
(74) Representative: Jackson, Derek Charles

(57) **Abstract**

A device for protecting the esophagus during a surgical ablation procedure within a heart of a patient comprises a catheter having a sensor (20, 60, 79, 80, 90) and positionable within an esophagus of a patient. An indicator (30, 62, 82, 106) is in communication with the sensor to indicate if a parameter exceeds a predetermined value, thereby indicating to the user to reduce or cease the ablation procedure within the heart of the patient.

## Description

### Technical Field

This application claims priority from provisional application serial no. 62/492,035, filed April 28, 2017, the entire contents of which is incorporated herein by reference.

This application relates to an esophageal retractor, and, more particularly, to an esophageal retractor with a sensor.

### Background Art

Atrial fibrillation is an irregular heart rhythm originating in the atrial (upper) chambers of the heart. In normal conditions, the heart is electrically excited to beat in a synchronous patterned fashion. In patients with cardiac arrhythmia, regions of the heart do not follow the synchronous beating cycle and instead aberrantly conduct to adjacent tissue, thereby disrupting the cardiac cycle into an asynchronous rhythm. Thus, atrial fibrillation is caused by an area within the heart producing an electrical disruption of the normal heart rhythm. The irregular rhythm causes palpitations and fatigue. The irregular rhythm also increases the risk of cerebral stroke as the abnormal wall motion of the left atrium can cause formation of thrombus within the atrial chamber, and such embolism can be pumped into the cerebral vessels. The irregular rhythm can also in some cases cause heart failure and even death.

One method to treat atrial fibrillation is ablation from inside the heart. To access the heart, catheters can be inserted in various ways including in an open procedure, thorascopically or minimally invasively through a remote access site into the left atrium. The catheter applies ablative energy such as radiofrequency, ultrasound laser energy, microwave energy, cryothermic energy, etc. The objective is to create a series of lesions along the cardiac tissue wall to create a barrier to propagation of the arrhythmia or to destroy an area of cardiac muscle to disrupt the arrhythmia. During the ablation procedure, there is a risk of damage to the esophagus do to its proximity to the heart, i.e., the posterior wall of the left atrium between the left and right pulmonary veins. In certain instances, conduction of heat from endocardial lesions can cause serious injuries to the esophagus such as acute-pyloric spasms, gastric hypomotility and can cause atrio-esophageal fistulas which can be fatal.

One attempt to avoid damage to the esophagus has been to reduce the power applied by the ablation device within the heart. However, this has the disadvantage of not forming the necessary lesions in the targeted area and thus not effectively treating atrial fibrillation due to inadequate energy application.

Another attempt to avoid damage to the esophagus has been to apply an esophageal cooling system. This requires a system for coolant delivery and for evacuation of the coolant.

Another attempt to protect the esophagus has been to monitor the esophageal temperature during the ablation procedure. However, these attempts do not necessarily provide an accurate reading of the area of the esophagus since the temperature sensor might not be positioned adjacent the heated area of the esophagus. Thus, a reading of one portion of the esophagus can be within acceptable limits while another region closer to the endocardial lesion can be outside the acceptable limits and suffer thermal damage. Further, since the esophagus is adjacent the heart, the temperature reading can occur too late in time. A device that better protects the esophagus during an ablation procedure within the heart treating atrial fibrillation would be advantageous.

The need therefore exists for an improved system to protect the esophagus during ablation procedures within the heart.

### Summary of invention

The present invention overcomes the deficiencies and disadvantages of the prior art. The present invention provides a system for accurate temperature monitoring or monitoring other parameters and for keeping the esophagus further spaced from the heart during ablation procedures within the heart. In some embodiments, the present invention provides an automatic system that is activated in response to temperature readings or reading of other parameters of the esophagus to minimize delay between a reading outside predetermined limits and steps to protect the esophagus during ablation procedure within the heart.

The present invention advantageously provides a minimally invasive device for detecting temperature rises in the esophagus due to heat from an ablation procedure wherein the temperature sensor is carried by an esophageal retractor. In some embodiments, the temperature sensor is tied into the ablation catheter to control ablation within the heart based on temperature measurements within the esophagus. In some embodiments, the temperature sensor is tied into the actuation of the esophageal retractor to move the esophagus away from the heated left atrium. The temperature sensor of the present invention can measure one or more of the fluid within a balloon of the esophageal retractor, the outer wall of the balloon of the esophageal retractor or the wall of the esophagus in order to provide an accurate reading for comparison to desired temperature limits to avoid thermal damage to the esophagus and the aforementioned consequences.

In accordance with one aspect, the present invention provides a device for protecting the esophagus during a surgical ablation procedure within a heart of a patient comprising a catheter having a temperature sensor, an indicator, a first lumen and a balloon in fluid communication with the first lumen and positionable within an esophagus of a patient. The temperature sensor measures one or more of a temperature of a fluid within the balloon, the temperature of the balloon or the temperature of the wall of the esophagus. The indicator is in communication with the sensor to indicate if the temperature of the fluid within the balloon exceeds a predetermined temperature, thereby indicating to the user to reduce or cease the ablation procedure within the heart of the patient.

In some embodiments, the balloon in an inflated condition has an asymmetrical configuration. In some embodiments, the temperature sensor is positioned internal of the balloon, in other embodiments the temperature sensor is positioned external of the balloon, and in other embodiments the temperature is sensor is embedded in a wall of the balloon.

In some embodiments, the indicator is an audible alarm. In other embodiments, the indicator is a visual indicator. In other embodiments, the indicator provides both a visual and an audible indication.

In some embodiments, the controller directly controls an ablation catheter operable to perform the ablation procedure within the heart. In some embodiments, the system includes a second controller, wherein the controller sends the signal to the second controller which then sends a signal to an ablation catheter within the heart to reduce or cease the application of ablation energy if the measured parameter exceeds the predetermined temperature.

The system can also include an ablation catheter for performing the ablation procedure within the heart.

In some embodiments, the parameter is measured during the procedure to provide a series of measurements to the controller.

In some embodiments, the parameter is temperature and if the temperature goes below the threshold after it was above the threshold, the controller sends a signal to resume the ablation procedure.

In accordance with another aspect, the present invention provides a system for protecting the esophagus during a surgical procedure comprising a device having a first lumen and a balloon in fluid communication with the first lumen, the device positionable within an esophagus of a patient. A sensor is carried by the device and measures a parameter. A controller is in communication with the sensor and in communication with an ablation catheter system positioned within the heart, the controller receiving a signal from the sensor representative of the measured parameter and comparing the measured parameter to a threshold value and if the measured parameter exceeds the threshold value, a signal is sent by the controller to the ablation catheter system to automatically reduce the energy applied by the ablation catheter to protect the esophagus.

In some embodiments, the energy is reduced by shutting off energy application.

In some embodiments, the parameter is measured during the procedure to provide a series of measurements to the controller.

In some embodiments, if the parameter goes below the threshold after exceeding the threshold, the controller sends a signal to resume the ablation procedure.

The sensor can be for example one or more of a temperature sensor, an electric field sensor, or a magnetic field sensor.

In accordance with another aspect, the present invention provides a device for protecting the esophagus during a surgical procedure comprising a first lumen, a balloon in fluid communication with the first lumen and positionable within an esophagus of a patient, and a temperature sensor to measure temperature during an ablation procedure in a heart of a patient. If the measured temperature exceeds a predetermined temperature a) an indicator is activated to alert the user and b) the balloon is automatically inflated to move esophagus away from the heart of the patient.

In some embodiments, the temperature sensor measures temperature of an outer wall of the balloon. In other embodiments, the temperature sensor measures temperature of fluid within the balloon. In other embodiments, the temperature sensor measures temperature of a wall of the esophagus.

In some embodiments, the balloon is inflated from a deflated condition. In other embodiments, the balloon is further inflated from a partially inflated condition.

In accordance with another aspect of the present invention, a method is provided for protecting the esophagus of the patient during an ablation procedure within the heart of the patient. The method comprises providing a catheter having a sensor, a first lumen and a balloon in fluid communication with the first lumen, inserting the catheter into the esophagus, measuring via the sensor a temperature of one or more of a fluid in the balloon, a surface of the balloon or a wall of the esophagus, and reducing ablation energy if a measured temperature exceeds a predetermined value.

In some embodiments, the method further comprises the step of inflating the balloon in response to the measured temperature exceeding the predetermined value to engage a wall of the esophagus and to move the esophagus away from the heart.

In some embodiments, if the measured temperature exceeds a threshold value, the balloon is automatically inflated to move the esophagus away from the heart. In some embodiments, the threshold value is the same as the predetermined value.

In some embodiments, the sensor is in communication with a controller and the controller sends a signal to automatically inflate the balloon if the measured temperature exceeds the predetermined value.

In some embodiments, inflating the balloon expands the balloon asymmetrically.

In some embodiments, the sensor is in communication with the controller, and the controller sends a signal to automatically reduce ablation energy applied to the heart if the measured temperature exceeds the predetermined value.

In some embodiments, an indicator is provided in communication with the sensor to indicate to a user that the temperature exceeds the predetermined value. In some embodiments, the indicator is a visual indicator and/or an audible indicator.

In the foregoing devices, systems and methods, sensors other than temperature sensors can be utilized such as electric field sensors and magnetic field sensors.

In accordance with another aspect, the present invention provides a system for protecting the esophagus during a surgical ablation procedure within a heart of a patient, the system comprising a catheter positionable within an esophagus of the patient. A sensor is carried by the catheter for measuring a parameter and an indicator is responsive to the sensor. A controller is in communication with the sensor and the indicator. The sensor sends a first signal to the controller indicative of the measured parameter and the controller compares the measured parameter to a predetermined value, wherein the system is configured such that in use if the measured parameter exceeds the predetermined value, the controller sends a signal to the indicator to alert a user that the measured parameter exceeds the predetermined value and optionally sends a signal to reduce or cease application of ablation energy within the heart. In such a case, the sensor may be a temperature sensor, a magnetic field sensor and/or an electric filed sensor. Further, the controller may be configured in use to send a signal to automatically a) reduce ablation energy applied to the heart and/or b) automatically inflate or further inflate a balloon to move the esophagus further away from the heart if the measured parameter exceeds the predetermined value.

### Brief description of drawings

So that those having ordinary skill in the art to which the subject invention appertains will more readily understand how to make and use the surgical apparatus disclosed herein, preferred embodiments thereof will be described in detail hereinbelow with reference to the drawings, wherein:
Figure 1A is a perspective view of a first embodiment of the esophageal retractor of the present invention with the balloon shown in the deflated (collapsed) condition;
Figure 1B is a perspective view similar to Fig. 1 showing the balloon in the inflated condition;
Figure 2A is a perspective view similar to Figure 1B showing an alternate embodiment of the present invention;
Figure 2B is a perspective view similar to Figure 1B showing another alternate embodiment of the present invention;
Figure 2C is a perspective view similar to Figure 1B showing another alternate embodiment of the present invention;
Figure 3 is a schematic view of one embodiment of the system of the present invention;
Figure 4 is a schematic view of another embodiment of the system of the present invention;
Figure 5 illustrates the system of Figure 4 showing the esophageal retractor within the esophagus prior to balloon inflation and further illustrating an ablation catheter within the heart;
Figure 6 is a view similar to Figure 5 illustrating the balloon inflated to move the esophagus away from the heart;
Figure 7 is a flow chart illustrating one method of the present invention;
Figure 8 is a flow chart illustrating another method of the present invention;
Figure 9 is a flow chart illustrating another method of the present invention;
Figure 10 is a flow chart illustrating another method of the present invention; and11 is a block diagram illustrating examples of various sensors used with the esophageal retractor.

### Description of embodiments

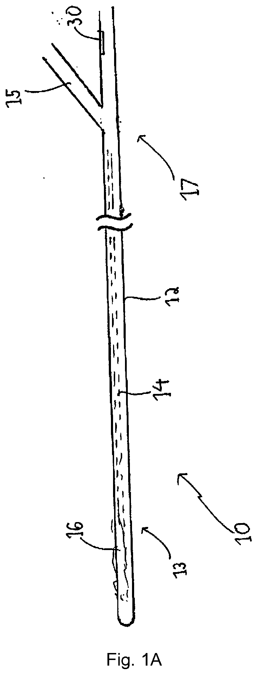

Referring now to the drawings wherein like reference numerals identify similar structural features of the device disclosed herein, there is illustrated in Figure 1A an esophageal retractor of the present invention. The esophageal retractor (device) is designated generally by reference numeral 10 and is configured for minimally invasive insertion into the esophagus of the patient. The retractor 10 is positionable within the esophagus for measuring temperature during an ablation procedure within the heart to treat atrial fibrillation. During such heart ablation procedures, the esophagus, which is positioned adjacent the heart, i.e., left atrium, can get overheated which can cause thermal damage to the esophagus and cause for example acute-pyloric spasms, gastric hypomotility, fatal atrio-esophageal fistulas, etc. as described above.

The esophageal retractor 10 of the present invention provides for inflation of the balloon to move the esophagus away from the heart plus monitors temperature to alert the user if the temperature rises to a level damaging to the esophagus. That is, although it is desirable to apply sufficient heat within the heart by an ablation catheter to ablate tissue and form lesions to treat conditions such as atrial ablation, such heating is not desirable for the esophagus. The present invention therefore is designed to keep the esophagus spaced from the heart and to alert the user if temperature within the esophagus rises to an unacceptable level, i.e., beyond a threshold or predetermined value (temperature). The temperature of the balloon, fluid within the balloon and/or the esophageal wall can be measured as described in detail below. In some embodiments, in addition to providing an alert to the user, the temperature monitoring automatically controls the ablation energy of the ablation catheter within the heart. In this manner, if the temperature exceeds a threshold level (value), the ablation energy can automatically be adjusted, i.e., reduced or terminated, to prevent overheating and damaging of the esophagus. In some embodiments, in addition to providing an alert to the user if temperature exceeds a predetermined threshold, the temperature monitoring automatically controls the inflation of the balloon to further its distance from the heart. In some embodiments, the temperature measurement is tied into both the balloon inflation and ablation energy control. These various embodiments are discussed in detail below.

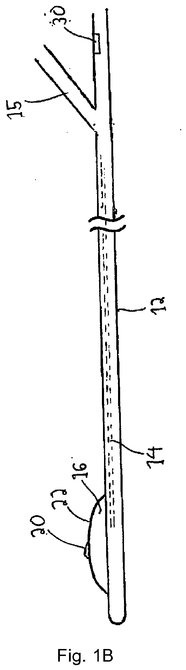

Turning now to details of the esophageal retractor 10 of the present invention, which is also referred to herein as the device 10 or retractor 10 or catheter 10, and with initial reference to Figures 1A and 1B, the retractor 10 has an elongated flexible shaft 12 having a lumen 14 extending within the shaft 12 and terminating at its distal end 13 at balloon 16 to fluidly communicate with balloon 16 to inflate the balloon. The shaft 12 is configured and dimensioned for minimally invasive insertion such as nasal or transoral insertion. A fluid side port 15 is positioned at a proximal region 17 of the retractor 10 for communication with an infusion source for infusion of fluid through the lumen 14 and into the balloon 16. The retractor 10 is shown in Figure 1A with balloon 16 in the deflated condition (position) and in Figure 1B with the balloon in the inflated condition (position). A temperature sensor 20, such as a thermocouple or thermistor, is positioned on an outer surface 22 of balloon 16. Note that although only one sensor is shown, it is also contemplated that multiple sensors can be provided. Also, although the sensor 20 is shown at a mid-portion of the balloon, it is also contemplated that the sensor can be placed at another portion, e.g., a distal portion, proximal portion, etc. Note if multiple sensors are provided, they can be positioned at various locations on the balloon.

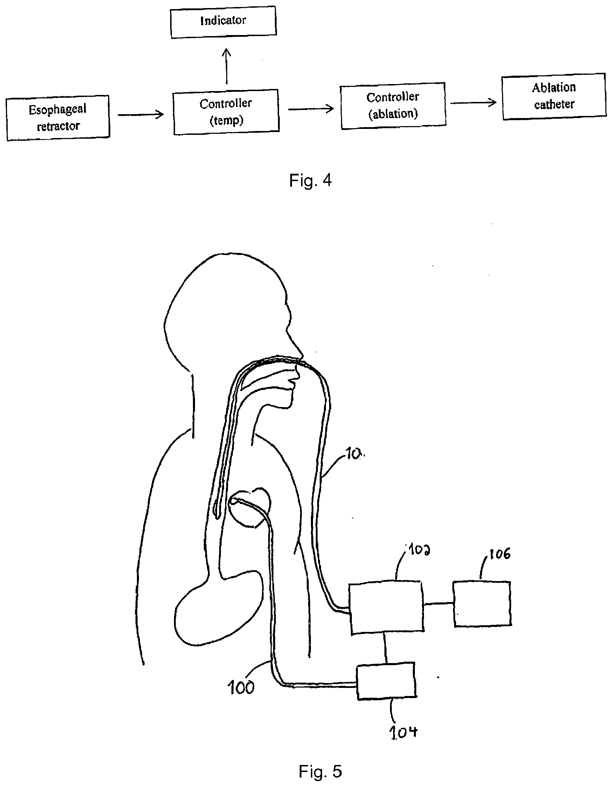

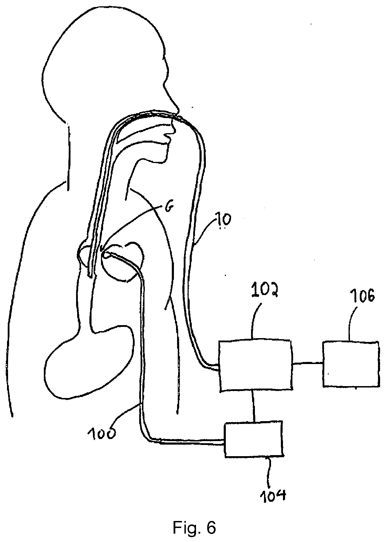

The balloon 16 as shown expands asymmetrically, i.e., to one side of the longitudinal axis of the retractor 10. The retractor 10 is positioned so the asymmetrical balloon faces away from the heart. In this manner, inflation of the balloon 16 presses the wall of the esophagus opposite the wall which is adjacent the heart, thereby applying a force on the wall that pushes the esophagus away from the heart. This can be appreciated by comparing Figure 5 where the esophagus is shown adjacent the heart wall and Figure 6 where the esophagus is shown moved away from the heart creating a gap G.

The sensor 20 is shown on an outer surface 22 of balloon 16. However, it is also contemplated that the sensor can be positioned adjacent the inner wall of the balloon 16 or alternatively embedded in the wall of the balloon 16. If multiple sensors are provided, one or more sensors can be at the external wall, the internal wall and/or embedded in the wall. In this embodiment of Figure 1B, the sensor 20 is designed to measure the temperature of the wall of the balloon 16. Thus, during an ablation procedure within the heart, as the heart wall is heated, heat is transmitted to the esophagus. The balloon 16 is inflated prior to or at the commencement or during the ablation procedure to retract the esophagus, and thus an outer surface of the balloon 16 is in contact with a wall of the esophagus. Therefore, heat would be transmitted to the balloon, and measuring the temperature of the balloon wall would provide an indication of the temperature of the esophageal wall and thus if the esophagus is at risk of thermal damage. The balloon 16 (as well as balloons 66, 76 and 86 discussed below) in some embodiments can be partially inflated to measure temperature (and move the esophagus wall) and if temperature rises past a predetermined level, further inflated to move the esophagus wall even further from the heart.

An indicator 30 in some embodiments is provided on the proximal portion of the retractor 10, preferably at a proximal end outside the patient's body. The sensor 20 is in communication with the indicator 30, either via connecting wires extending through a lumen of the retractor 10, wires extending alongside the retractor 10, or via a wireless connection. The sensor 20 in some embodiments is part of a system that includes a comparator so that a comparison of the measured temperature to a predetermined threshold temperature value is performed within the retractor 10 and a signal is sent to the indicator 30 to activate (actuate) the indicator 30 if the measured temperature exceeds the threshold temperature to alert the user that the temperature within the esophagus is too high so appropriate steps can be taken to protect the esophagus. If the measured temperature is below the threshold, the indicator 30 is not activated and the ablation procedure within the heart can continue uninterrupted. The indicator can be a visual indicator and/or an audible i nd icator.

In other embodiments, the sensor 20 carried by the retractor 10 is connected to a controller positioned outside the body via wires or a wireless connection. The sensor 20 sends a signal representative of the measured temperature of the balloon wall wherein the measured temperature is compared by the controller, e.g., a comparator, to determine if it is above a threshold (predetermined) temperature (value). If it is below a threshold value, then the indicator 30, electrically connected to the controller via wire connection or wireless connection, is not actuated (activated) and the ablation procedure within the heart can continue uninterrupted. However, if the measured temperature is above the threshold value, a signal is sent by the controller to the indicator 30 to alert the user that the temperature within the esophagus is too high and that steps need to be taken to reduce or cease (terminate) the ablation energy applied by the ablation catheter within the patient's heart. The controller can send a signal to the indicator which is located on the catheter. Alternatively, the indicator can be positioned on the controller, e.g., on a screen or panel, or on a separate display outside the patient, to alert the user.

Figure 5 shows another alternative embodiment of the indicator where the system includes an indicator as a separate unit (component) in electrical communication with the controller. Features of this system are described in detail below.

The indicator 30 in the various embodiments described herein can be a visual indicator such as a light, LED, color change, etc. Alternatively, or additionally, the indicator can be an audible indicator which emits some type of sound or alarm to alert the user. Note the controller and/or the indicator in some embodiments can also display the temperature reading e.g., as a numeric or graphical value.

The indicator 30 is shown at the proximal region of the retractor 10 in Figures 1A and 1B, but it is also contemplated that the indicator could be positioned at other portions of the retractor 10, e.g., at a distal end portion, where known imaging techniques would enable the user to discern when the visual indicator is turned on. Audible indicators could likewise be positioned at other regions of the retractor 10.

The temperature can be measured by one or more sensors 20 intermittently, e.g., at spaced time intervals, or continuously during the entire heart ablation procedure to provide respective periodic or continuous temperature measurement/readings for comparative analysis to the threshold value so that ablation can be monitored and controlled at various times or throughout the ablation procedure.

In some embodiments, the sensor is tied into automatic control of the ablation catheter. This alternate embodiment is discussed in detail below. In some embodiments, the sensor is tied into balloon inflation. This is also discussed in detail below.

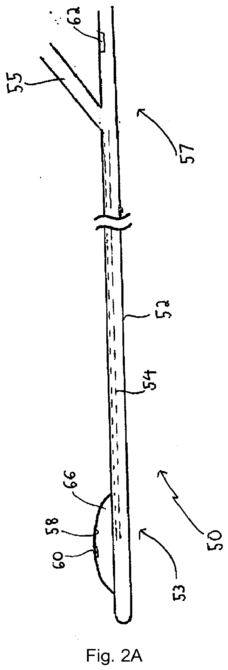

Figure 2A illustrates an alternate embodiment of the esophageal retractor of the present invention. Retractor 50 is identical to retractor 10 except for the location and function of the sensor. More specifically, retractor 50 has an elongated flexible shaft 52 like the elongated flexible shaft 12 of retractor 10 configured for minimally invasive, e.g., transoral or nasal insertion, a lumen 54 extending within the shaft 52 and terminating at is distal end 53 within balloon 66 to fluidly communicate with balloon 66, and a fluid side port 55 positioned at a proximal region 57 of the retractor 50 for communication with an infusion source for infusion of fluid through the lumen 54 and into the balloon 66. A sensor 60 is positioned within the balloon 66, and in the illustrated embodiment, on an internal wall 58 of the balloon 66. Alternatively, the sensor can be embedded in a wall of the balloon or on an exterior wall of the balloon. If multiple sensors are provided, one or more sensors can be at the external wall, the internal wall or embedded in the wall. In this embodiment, the sensor 60 measures temperature of the fluid within the balloon. Thus, during an ablation procedure within the heart, as the heart wall is heated, heat is transmitted to the esophagus. The balloon 66 is inflated to retract the esophagus, prior to, at the commencement of or during ablation procedure. The balloon 66 and fluid inside the balloon 66 heats up as the balloon is in contact with a wall of the esophagus. Therefore, heat would be transmitted to the balloon 66 and the fluid within the balloon 66, and measuring the temperature of the fluid would provide an indication of the temperature of the esophageal wall and thus if the esophagus is at risk of thermal damage. The sensor 60 is in communication with an indicator 62 on the retractor 50. Alternatively, it can be in communication with a controller. The connection/communication can be via wires extending adjacent or through the retractor 10 or wireless. The aforedescribed various types of sensors, locations of sensor 20 and its various connections to the components, e.g., part of the controller or separate component, are fully applicable to the sensor 60 and therefore for brevity these various alternatives/embodiments are not repeated herein. Likewise the various types, connections and locations of indicator 30 are fully applicable to the indicator 62 (e.g., on the catheter, external, separate display, etc) and therefore for brevity are not repeated herein.

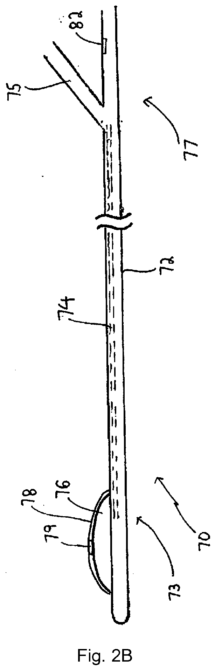

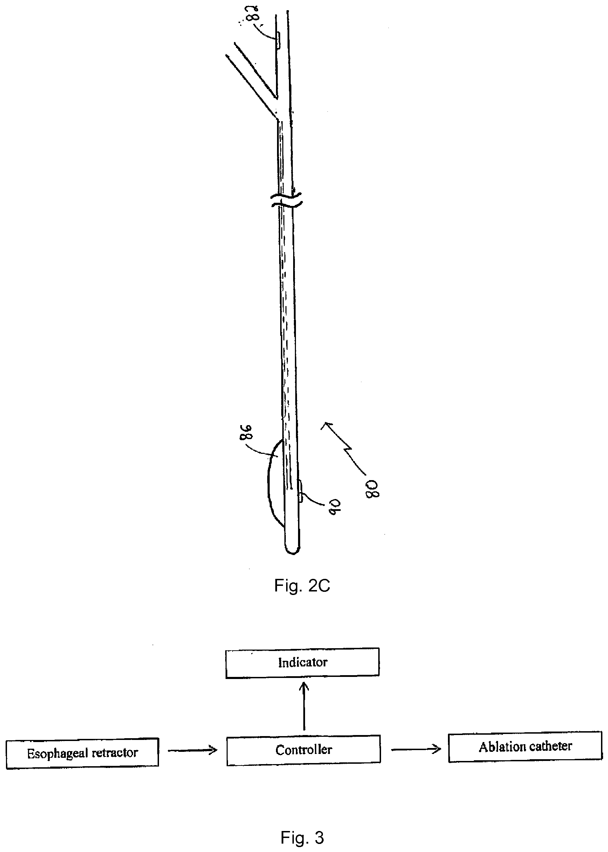

Figure 2B illustrates an alternate embodiment of the esophageal retractor of the present invention. Retractor 70 is identical to retractor 10 except for the location and function of the sensor. More specifically, retractor 70 has an elongated flexible shaft like the elongated flexible shaft 12 of retractor 10 configured for minimally invasive, e.g., nasal or transoral insertion, a lumen 74 extending within the shaft 72 and terminating at is distal end 73 within balloon 76 to fluidly communicate with balloon 76, and a fluid side port 75 positioned at a proximal region 77 of the retractor 70 for communication with an infusion source for infusion of fluid through the lumen 74 and into the balloon 76 to inflate the balloon 76. A sensor 79 is embedded in the wall 78 of the balloon 76. Alternatively, the sensor can be on an external surface of the wall of the balloon or on an interior wall of the balloon. The sensor can also be located on the retractor 10 in a region opposite the balloon 76, i.e., on the side of the retractor that is adjacent the side of the esophageal wall that is closer to the heart as shown in Figure 2C. The sensor can also be located on the device 10 at a region spaced from the balloon 76, e.g., proximal or distal of the balloon 76. If multiple sensors are provided, one or more sensors can be at the external wall, the internal wall, embedded in the wall, on a region of the catheter spaced from the wall, etc. In this embodiment, the sensor 80 measures temperature of the esophageal wall. During an ablation procedure within the heart, as the heart wall is heated, heat is transmitted to the esophagus. The balloon 76 is inflated prior to, at the commencement of or during the ablation procedure. The sensor 79 can measure the temperature of the esophageal wall adjacent the balloon 76. Since the inflated balloon pushes a portion of the wall of the esophagus further from the heart, the sensor on the balloon would measure the esophageal temperature on the further wall of the esophagus. In alternate embodiments, the sensor is on the retractor at the distal end in the same region as the balloon, e.g., aligned with the balloon, but on an opposite side of the longitudinal axis of the retractor 80. This is shown in the embodiment of Figure 2C which is identical to the embodiment of Figure 2B except for the location of the sensor 90 for measuring temperature of the esophageal wall. In this manner, the portion of the esophageal wall closer to the heart is measured by the sensor 90. That is, the retractor 80 is positioned in the esophagus so the sensor 90 faces the heart and the balloon 86 faces away from the heart. The balloon 86 of retractor 80 is inflated to retract the esophagus. The balloon 86 can be inflated prior to, at the commencement or during the ablation procedure. Since the sensor 90 is independent of balloon movement the balloon 86 could alternatively remain deflated until the esophageal wall temperature exceeds a predetermined value where the balloon 86 would then be inflated, either by action by the user or automatically if inflation is electrically tied into temperature measurement, to move the esophageal wall away from the heart to reduce heat transfer to the esophageal wall.

Measuring the temperature of the esophageal wall will enable assessment if the esophagus is at risk of thermal damage. The sensor 80 (or sensor 90) is in communication with an indicator 82 on the retractor 70 (or 80). Alternatively, it can be in communication with a controller as described above with respect to the other embodiments. The connection/communication can be via wires extending through or adjacent the retractor 70 (or 80) or can be a wireless connection. The aforedescribed various types of the sensor and indicator, locations of the sensor and indicator, and its various connections to the components are fully applicable to the sensor 80 and sensor 90 and the indicator 82 and therefore for brevity these various alternatives/embodiments are not repeated herein.

Turning now to details of the system, Figure 3 illustrates schematically one embodiment of the system wherein the temperature measurement system is tied into the heart ablation system. In this system, the esophageal retractor measures the temperature using one or more of the methods described above, and transmits a signal representative of the measured temperature to a controller. The controller compares the measured temperature to a threshold (predetermined) value and if the temperature exceeds the threshold value, the controller 1) sends a signal to the indicator to alert the user that the temperature threshold is exceeded and 2) sends a signal to the ablation catheter to regulate the application of ablation energy of the ablation catheter, i.e., reduce the power/energy applied or if necessary terminate the energy delivery. The temperature can be subsequently monitored, either intermittently or continuously, to provide subsequent temperature measurements. In this manner, if the energy was reduced in response to the controller, and a subsequent temperature measurement indicates sufficient cooling, the energy level can be raised or energy reapplied to continue the ablation procedure. Also, in this manner, if the temperature rises after an initial reading indicated it was below the threshold, power/energy can be reduced.

As can be appreciated, in the embodiment of Figure 3, a single controller is utilized for comparing temperature measurements and for regulating the ablation catheter. In the alternate embodiment depicted schematically in Figure 4, a separate controller for the ablation catheter is utilized. This is also shown in Figure 5 wherein the ablation catheter 100 is connected to another controller 104 which regulates application of energy, e.g., an RF generator, and the esophageal retractor of the present invention, e.g., retractor 10, although other retractors disclosed herein can be utilized, is connected to controller 102. Controller 102 is connected to indicator 106, e.g. an external indicator which provides an alert if temperature of the esophagus is exceeded. Thus, controller 102 conducts the comparison of measured temperature and sends a signal to controller 104 which regulates the power applied in accordance with the received signal. Controller 102 can include indicators to indicate the energy applied or a separate indicator component can be utilized such as schematically depicted as indicator 106. As can be appreciated, both the system of Figure 3 and the system of Figure 4 enable automatic adjustment of the ablation procedure to provide automatic heat reduction of the esophagus, saving reaction time to esophagus heating since the controller can respond more quickly than a clinician in adjusting power.

Note that in embodiments with multiple sensors, average temperatures can be computed and compared to a threshold value or each temperature measurement can be analyzed separately and compared to the threshold value to assess risk of thermal damage to the esophagus.

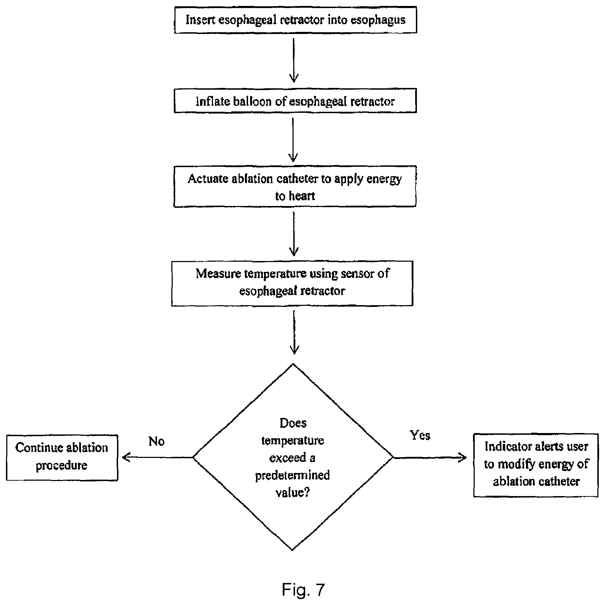

Turning now to the method of use of the retractor and systems of the present invention, these various systems are depicted in the flow charts of Figures 7-10.

With initial reference to the system of Figure 7, in the first step, the esophageal retractor is inserted into the esophagus in a minimally invasive manner with the balloon in the deflated condition. The retractor is advanced to a region of the esophagus adjacent the region of the heart which is the target of ablation energy to form lesions. (Such positioning is shown in Figure 5). Next, the balloon is inflated, expanding to one side of the retractor. The retractor is oriented so that balloon expansion moves the esophageal wall which is adjacent the heart in a direction away from the heart to create a gap to reduce heat conduction. (Such movement of the esophageal wall is shown in Figure 6). Note the retractor can have markings or indicators to help the user orient the retractor so the balloon faces away from the esophageal wall adjacent the heart. Next, the ablation catheter which is positioned within the heart is activated to apply ablation energy to a wall of the heart, e.g., a left atrium, to form lesions to treat atrial fibrillation. The sensor(s) carried by the esophageal retractor measures one or more of the temperature of the balloon wall, fluid within the balloon and/or the esophagus as described above. The measured temperature is compared to a predetermined value. If the measured temperature is below the predetermined value, the ablation procedure is continued. However, if the measured temperature is above the predetermined (threshold) value, the indicator is activated to alert the user that the temperature is too high and steps need to be taken to protect the esophagus from thermal damage. The user can then reduce or cease application of energy to the ablation catheter. Note, in some embodiments, the balloon is partially inflated during initial temperature measurement to slightly move the esophagus away from the heart and if temperature rises to a predetermined level, which could be less than or equal to the level which triggers the indicator, the balloon can be further inflated to move the esophagus further away from the heart to reduce heat transfer.

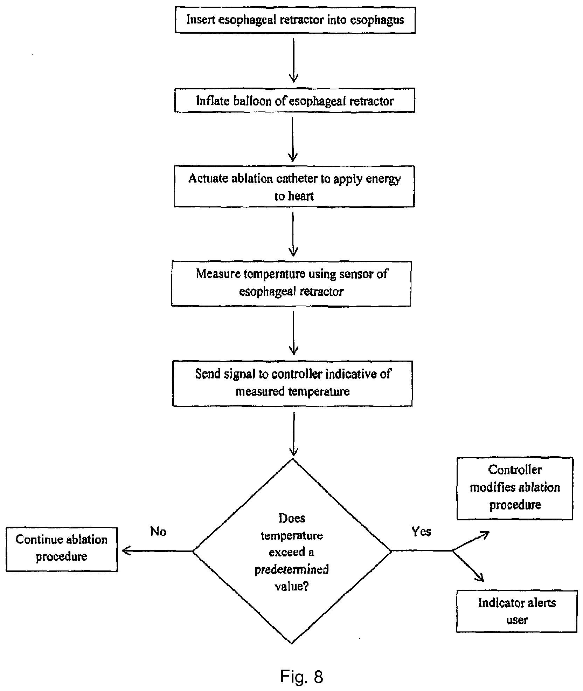

The alternate system depicted in the flow chart of Figure 8 is similar to Figure 7 except the power supplied by the ablation catheter is tied into temperature measurement of the catheter and automatically regulated. More specifically, like the system of Figure 7, in the first step, the esophageal retractor is inserted into the esophagus in a minimally invasive manner with the balloon in the deflated condition. The retractor is advanced to a region of the esophagus adjacent the region of the heart which is the target of ablation energy to form lesions. Next, the balloon is inflated, expanding to one side of the retractor. The retractor is oriented so that balloon expansion moves the esophageal wall which is adjacent the heart in a direction away from the heart. Next, the ablation catheter which is positioned within the heart is activated to apply ablation energy to a wall of the heart, e.g., a left atrium, to form lesions to treat atrial fibrillation. The sensor(s) carried by the esophageal retractor measures one or more of the temperature of the balloon wall, fluid within the balloon and/or the esophagus as described above. The sensor sends a signal to the controller indicative of the measured temperature. The measured temperature is compared to a predetermined value. If the measured temperature is below (does not exceed) the predetermined value, the ablation procedure is continued. However, if the measured temperature is above the predetermined value, the indicator is activated to alert the user that the temperature is too high and the controller, either a separate second controller linked (in electrical communication) to the first controller and the ablation catheter or a single controller that is linked (in electrical communication) to both the retractor sensor and the ablation catheter, automatically adjusts, i.e., lowers or terminates, the power applied by the ablation catheter to lower the heat conduction of the heart wall and thus lower the heat applied and temperature of the esophageal wall to protect the esophagus from thermal damage. When the temperature of the esophagus reduces to an acceptable value, the ablation procedure can be resumed.

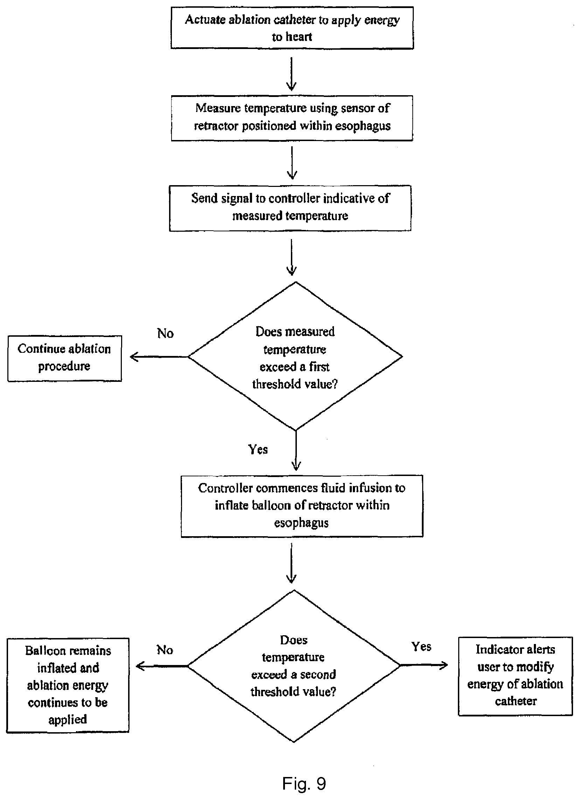

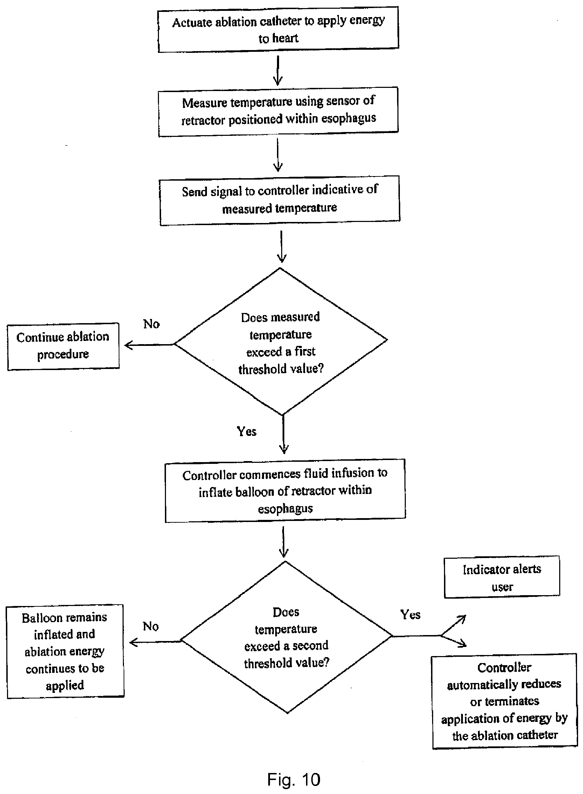

Figures 9 and 10 provide flow charts depicting alternate systems of the present invention wherein temperature measurement is tied into balloon inflation. In the first step, like in Figure 8, the esophageal retractor is inserted into the esophagus in a minimally invasive manner with the balloon in the deflated (or partially inflated) condition. The retractor is advanced to a region of the esophagus adjacent the region of the heart which is the target of ablation energy to form lesions. (Such positioning is shown in Figure 5). The retractor is oriented so that balloon expansion can move the esophageal wall which is adjacent the heart in a direction away from the heart to create a gap to reduce heat condition, but the balloon is not yet inflated or is only partially inflated. As shown in Figure 9, next the ablation catheter which is positioned within the heart is activated to apply ablation energy to a wall of the heart, e.g., a left atrium, to form lesions to treat atrial fibrillation. The sensor(s) carried by the esophageal retractor measures one or more of the temperature of the balloon wall, fluid within the balloon and/or the esophagus as described above. The measured temperature is compared to a first predetermined (threshold) value. If the measured temperature is below the predetermined value, the ablation procedure is continued and the balloon is not inflated (or further inflated), leaving the esophagus in position. However, if the measured temperature is above the first predetermined value, the indicator is activated to alert the user that the temperature is too high and the balloon is automatically inflated to move the esophagus away from the heart. A visual or audible indicator can be provided to indicate such temperature level has been reached and activation of balloon inflation. This position is shown in Figure 6. That is, the controller in this embodiment controls the flow of inflation fluid into the balloon such that if needed based on temperature rising above the threshold value, fluid is inserted (injected) within the balloon to inflate the balloon to move the esophagus to protect it against thermal damage. Thus, in this embodiment, balloon expansion occurs only if necessary therefore only exerting an expansion on and movement of the esophageal wall if necessary to protect the esophagus from thermal damage. If further protection is needed after movement of the esophageal wall away from the heart, the user can reduce or cease application of energy to the ablation catheter. The system in Figure 9 results in the esophagus being expanded a reduced amount of time. Note prior to the activation of the balloon in response to temperature measurement, the balloon can be in a fully deflated position or in a partially inflated position. After the balloon is inflated (or further inflated) to move the esophagus in response to the increased temperature, temperature measurement continues as the sensor sends signals to the controller. If temperature exceeds a second predetermined value, higher than the first predetermined value which triggers balloon inflation, the indicator is activated to alert the user that the temperature is too high and ablation energy is reduced or terminated. Note in alternate embodiments the first predetermined value can be equal to the second predetermined value so the indicator and fluid infusion occur at the same time (value). The controller communicating with the sensor could be the same controller that controls infusion (expansion) of the balloon in response to temperature measurement, or alternatively, the controller for balloon expansion can be a separate controller from the controller linked to the temperature indicator.

In the system depicted in the flow chart of Figure 10, temperature measurement is tied into balloon inflation as well as ablation energy application. As in the first four steps of the system of Figure 9, the esophageal retractor is inserted into the esophagus in a minimally invasive manner with the balloon in the deflated (or partially inflated) condition. The retractor is advanced to a region of the esophagus adjacent the region of the heart which is the target of ablation energy to form lesions. (Such positioning is shown in Figure 5). The retractor is oriented so that balloon expansion can move the esophageal wall which is adjacent the heart in a direction away from the heart to create a gap to reduce heat conduction, but the balloon is not yet inflated or is only partially inflated. Next, the ablation catheter which is positioned within the heart is activated to apply ablation energy to a wall of the heart, e.g., a left atrium, to form lesions to treat atrial fibrillation. The sensor(s) carried by the esophageal retractor measures one or more of the temperature of the balloon wall, fluid within the balloon and/or the esophagus as described above. The measured temperature is compared to a first predetermined (threshold) value. If the measured temperature is below the predetermined value, the ablation procedure is continued and the balloon is not inflated (or further inflated), leaving the esophagus in position. However, if the measured temperature is above the first predetermined value, the balloon is automatically inflated to move the esophagus away (or further away) from the heart. An audible or visual indicator can be provided to indicate such temperature level reached and activation of balloon inflation. This positon is shown in Figure 6. That is, the controller in this embodiment controls the flow of inflation fluid into the balloon such that if needed based on temperature rising above the threshold value, fluid is injected into the balloon to inflate the balloon to move the esophagus to protect it against thermal damage. Thus, in this embodiment, balloon expansion occurs only if necessary, therefore only exerting an expansion force on and movement of the esophageal wall if necessary to protect the esophagus from thermal damage. This also results in the esophageal wall being expanded a reduced amount of time. Note prior to the alert and activation of the balloon, the balloon can be in a fully deflated position or in a partially inflated position. After the balloon is inflated (or further inflated) to move the esophagus in response to the increased temperature, temperature measurement continues as the sensor sends signals to the controller. If temperature exceeds a second predetermined value, higher than the first predetermined value which triggers balloon inflation, the indicator is activated to alert the user that the temperature is too high and ablation energy is reduced or terminated. Note in some embodiments the first predetermined value can be equal to the second predetermined value so the indicator and fluid infusion occur at the same time (value). The controller communicating with the sensor and retractor could be the same controller that controls infusion (expansion) of the balloon in response to temperature measurement, or alternatively, the controller for balloon expansion can be a separate controller from the controller linked to the temperature indicator.

Additionally, in this embodiment of Figure 10, temperature measurement is also tied into regulation of ablation energy. If the measured temperature is below the second predetermined value, the ablation procedure is continued. However, if the measured temperature is above the second predetermined value, the indicator is activated to alert the user that the temperature is too high and the controller, either a separate controller in communication with the ablation catheter or a single controller that is linked to (in communication with) both the retractor sensor and the ablation catheter, automatically adjusts, i.e., lowers or terminates, the power applied by the ablation catheter to lower the heat conduction of the heart wall and thus lower the heat applied and temperature of the esophageal wall to protect the esophagus from thermal damage. When the temperature of the esophagus reduces to an acceptable value, the ablation procedure can be resumed. Note the indicator for excess temperature to activate balloon inflation (exceeding first predetermined level) can be the same indicator for energy adjustment (exceeding the second predetermined level) or alternatively a separate indicator, i.e. one indicator for activation of the balloon and one indicator for energy adjustment. Also, the predetermined (threshold) value for energy adjustment, i.e., reduction or cessation, can be the same threshold value as for balloon inflation or, as depicted in Figure 10, it can be a second different predetermined value, such that one value triggers balloon inflation and another value, e.g. a higher value, triggers energy reduction.

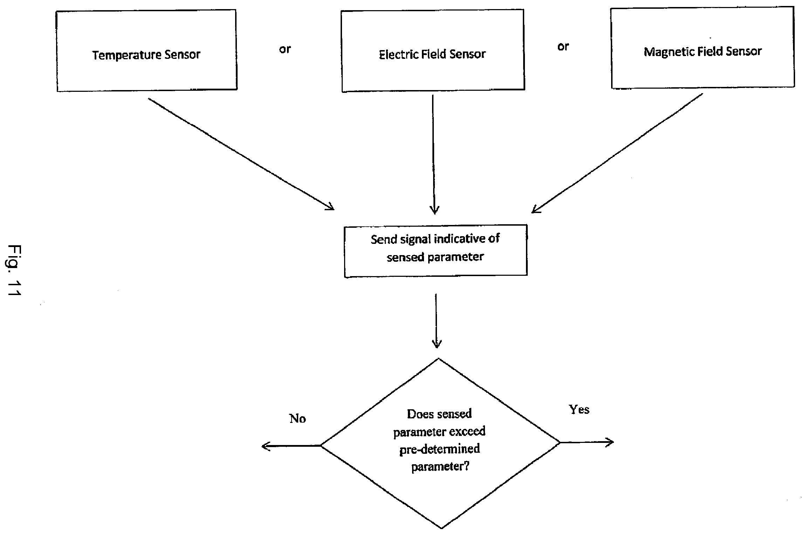

As an alternative to temperature sensors, electric field proximity sensors which detect changes in an electromagnetic field or magnetic field sensors (MEMS) which can measure change in voltage or frequency or displacement can be utilized. Figure 11 is a block diagram illustrating these alternatives. In the systems and methods described herein and depleted in the flowcharts, instead of the temperature sensor and temperature values to activate control the indicator, balloon inflation and/or ablation energy, these other sensors would be utilized and the measured parameter compared to a predetermined (threshold) parameter(s) to activate control the indicator, balloon inflation and/or ablation energy. Thus, Figure 11 illustrates how the sensed parameter (or value) is compared to a predetermined parameter (or value). The remaining steps would be the same as in the flow charts of Figures 7-10. That is, the flow charts of Figures 7-10 are fully applicable to these other sensors, the only difference being that electric field or magnetic field would be the basis for measurement rather than temperature. It should be appreciated that a combination of the types of sensors depicted in Figure 11 can be utilized with the esophageal retractor.

The balloons disclosed here are asymmetric with an arcuate semi-oval shape, however, balloons of different configurations could also be utilized. Additionally, more than one balloon carrying one or more sensors could be provided.

The retractors (catheter) disclosed herein have a lumen for balloon inflation. The retractors could have additional lumens for other functions.

A display can be provided separate from the retractor to show the measured temperature throughout the procedure. The display can be in addition or an alternative to an audible or visual alarm indicator which indicates excessive temperature.

As an alternative to balloons, a mechanical retractor having a sensor such as a sensor of Figure 11 mounted thereon could be utilized. For example, an actuator would be connected to the retractor and moved axially to expand links of the retractor.

Although the apparatus and methods of the subject invention have been described with respect to preferred embodiments, those skilled in the art will readily appreciate that changes and modifications may be made thereto without departing from the spirit and scope of the present invention as defined by the appended claims.

## Claims

1. A device for protecting the esophagus during a surgical ablation procedure within a heart of a patient, the device comprising a catheter having a temperature sensor (20, 60, 79, 80, 90) for measuring temperature, an indicator (30, 62, 82, 106), a first lumen (14, 54, 74) and a balloon (16, 66, 76, 86) in fluid communication with the first lumen and positionable within an esophagus of a patient, the indicator being in communication with the sensor to indicate if the temperature exceeds a predetermined temperature, thereby indicating to the user to reduce or cease the ablation procedure within the heart of the patient.

2. A device as claimed in claim 1, wherein the balloon (16, 66, 76, 86) in an inflated condition has an asymmetrical configuration.

3. A device as claimed in claim 1 or 2, wherein the temperature sensor (60) is positioned internal of the balloon (66) to measure temperature of a fluid within the balloon.

4. A device as claimed in claim 1 or 2, wherein the temperature sensor (20) is positioned to measure temperature of an outer wall of the balloon (16) and the sensor is configured to indicate if the temperature of the wall of the balloon exceeds the predetermined temperature

5. A device as claimed in claim 1, 2 or 4, wherein the sensor (79) is embedded in a wall of the balloon (76).

6. A device as claimed in claim 1 or 2, wherein the temperature sensor (80, 90) is positioned on the device to measure in use temperature of a wall of the esophagus, the indicator (82) configured to indicate if the temperature of the wall of the esophagus exceeds a predetermined temperature.

7. A device as claimed in any preceding claim in combination with a controller (102) in communication with the indicator (106) and sensor, the sensor configured to send a signal to the controller indicative of the measured temperature and the controller configured to send a signal to the indicator if the measured temperature exceeds the predetermined temperature to alert the user.

8. A device as claimed in claim 7, wherein the controller (102) is configured to directly control an ablation catheter (100) operable to perform the ablation procedure within the heart and if the measured temperature exceeds the predetermined temperature, the controller is configured to send a signal to automatically reduce or cease application of ablation energy within the heart to protect the esophagus.

9. A device as claimed in claim 7, further comprising a second controller (104), wherein the controller (102) is configured to send the signal to reduce or cease application of ablation energy to the second controller which is configured then to send a signal to an ablation catheter (100) within the heart to reduce or cease the application of ablation energy if the measured temperature exceeds the predetermined temperature.

10. A device as claimed in claim 7, 8 or 9, in combination with an ablation catheter (100) for performing the ablation procedure within the heart.

11. A device as claimed in any preceding claim, wherein the device is configured in use to automatically inflate the balloon (16) to move the esophagus away from the heart if the measured temperature exceeds a threshold value.

12. A device as claimed in claim 11, wherein the threshold value is the same as the predetermined temperature.

13. A system for protecting the esophagus during a surgical ablation procedure within a heart of a patient, the system comprising: a device as claimed in any preceding claim; and a controller (102) in communication with the temperature sensor (20, 60, 79, 80, 90) and the indicator (30, 62, 82, 106), the sensor for sending a first signal to the controller indicative of the measured parameter and the controller for comparing the measured parameter to a predetermined value, wherein if the measured parameter exceeds the predetermined value, the controller is configured to send a signal to the indicator to alert a user that the measured parameter exceeds the predetermined value.

14. A system as claimed in claim 13, wherein the controller (102) is configured in use to send a signal to automatically a) reduce ablation energy applied to the heart and/or b) automatically inflate or further inflate the balloon (16, 66, 76, 86) to move the esophagus further away from the heart if the measured parameter exceeds the predetermined value.
